Europäisches Patentamt

(19) European Patent Office

Office européen des brevets

(11) Veröffentlichungsnummer: **0 062 148**
A1

(12) **EUROPÄISCHE PATENTANMELDUNG**

(21) Anmeldenummer: **82101077.4**

(22) Anmeldetag: **13.02.82**

(51) Int. Cl.³: **A 61 M 1/03**, A 61 M 25/00

(30) Priorität: **07.04.81 DE 3113934**

(43) Veröffentlichungstag der Anmeldung: **13.10.82**
**Patentblatt 82/41**

(84) Benannte Vertragsstaaten: **AT BE CH DE FR GB IT LI LU NL SE**

(71) Anmelder: **Laboratorien Hausmann AG, Rechenstrasse 37, CH-9001 St. Gallen (CH)**

(72) Erfinder: **Berger, Franz, Lerchenstrasse 31, CH-9202 Gossau (CH)**

(74) Vertreter: **Gille, Christian, Dipl.-Ing. et al, Redies, Redies, Türk & Gille, Patentanwälte Brucknerstrasse 20, D-4000 Düsseldorf 13 (DE)**

(54) **Vorrichtung zur kontinuierlichen ambulanten Peritoneal-Dialyse.**

(57) Die Vorrichtung zur kontinuierlichen ambulanten Peritoneal-Dialyse umfaßt einen flexiblen Behälter (1) für die Dialyse-Flüssigkeit, ein Überleit-Schlauchsystem (21), einen Katheter (10) und Kupplungen (2, 5 und 6, 9) sowohl zwischen dem Überleit-Schlauchsystem (21) und dem Behälter (1) als auch zwischen dem Überleit-Schlauchsystem (21) und dem Katheter (10), wobei die Kupplungsstücke (2, 5, 6) am Überleit-Schlauchsystem (21) und Behälter (1) mittels Schutzkappen (8, 17, 18) vor Gebrauch steril abdeckbar sind. Die zusammengesteckten Kupplungen (2, 5 und 6, 9) können mittels einer Schutzkapsel (12) oder einer Schrumpfhülle (11) abgedeckt werden.

Laboratorien Hausmann AG,
Rechenstraße 37, CH-9001 St. Gallen, Schweiz

Vorrichtung zur kontinuierlichen ambulanten Peritoneal-Dialyse

Die Erfindung betrifft eine Vorrichtung zur kontinuierlichen ambulanten Peritoneal-Dialyse (CAPD), mit einem flexiblen Behälter für die Dialyse-Flüssigkeit, einem Überleit-Schlauchsystem und einem abkuppelbaren Katheter, das mittels einer chirurgischen Operation in die Bauchhöhle des Patienten einzulegen ist.

Die CAPD ist ein zur konventionellen Hämodialyse alternatives Verfahren zum Entgiften des Körpers von Patienten mit gestörter Nierenfunktion. In Phasen einer ungenügenden oder total ausfallenden Nierenfunktion kann der Mensch nur mittels Hämodialyseverfahren am Leben gehalten werden.

Bekannte Vorrichtungen für die CAPD haben einen die sterile Dialyselösung enthaltenden Behälter und ein Überleitsystem, mit welchem die sterile Dialyselösung unter peinlichster Vermeidung einer Kontamination mit Mikroorganismen, Viren oder Fremdpartikeln über ein in die Bauchwand des Patienten einoperiertes Katheter in die Bauchhöhle eingebracht und nach der gewünschten Verweilzeit wieder aus der Bauchhöhle entfernt werden kann.

Während das Problem einer leicht handhabbaren und die Bewegungsfreiheit des Patienten nicht einschränkenden Vorrichtung mittels flexibler beutelartiger Formkörper aus geeigneten Kunststoffen für den Behälter relativ einfach zu lösen war, verursacht die Aufrechterhaltung der Sterilität des gesamten Systems in allen Applikationsphasen, die täglich viermal wiederholt werden müssen, außerordentliche Schwierigkeiten. Schon eine durch einen einzigen Manipulationsfehler auftretende geringfügige Kontamination mit Luftkeimen führt fast unvermeidlich zu einer Bauchfellentzündung (Peritonitis). Daher kommt der Aufrechterhaltung der Sterilität der gesamten Vorrichtung in allen Applikationsphasen und der sicheren Vermeidung von Kontaminationsrisiken bei allen Kupplungs- und Entkupplungsvorgängen für eine erfolgreiche CAPD entscheidende Bedeutung zu.

Der Erfindung liegt die Aufgabe zugrunde, eine Vorrichtung zur kontinuierlichen ambulanten Peritoneal-Dialyse (CAPD) zu schaffen, bei der der Behälter für die Dialyse-Lösung an das für die Überführung dieser Lösung verwendete Überleit-Schlauchsystem auch von weniger geschulten Personen und auch in normaler Umgebung ohne irgendwelche Kontaminationsrisiken an- bzw. abgekuppelt werden kann, beispielsweise vom Patienten selbst in seiner normalen Wohnumgebung, und bei welchem auch die von medizinischen Fachpersonen vorzunehmende Verbindung zwischen dem Überleit-Schlauchsystem mit dem Katheter ohne Kontaminationsgefahr hergestellt werden kann.

Diese Aufgabe wird bei einer Vorrichtung der eingangs genannten Gattung erfindungsgemäß mit den Merkmalen des kennzeichnenden Teils des Hauptanspruches gelöst. Vorteilhafte Ausgestaltungen der Erfindungen sind Gegenstand der Unteransprüche.

Bei der CAPD läßt der Patient innerhalb von vierundzwanzig Stunden viermal je zwei Liter Dialyse-Lösung in die Bauchhöhle einfließen, wobei während des Tages die eingebrachte Lösung nach fünf Stunden, in der Nacht nach neun Stunden aus der Bauchhöhle abfließen gelassen wird. Nach jeder Entleerung werden sofort wieder zwei Liter frische Dialyse-Lösung einfließen gelassen. Das in die Bauchhöhle des Patienten einoperierte, mit einem Anschlußkupplungsstück versehene Katheter kann jahrelang im Patienten verbleiben. Das zum Überleiten verwendete Schlauchsystem wird alle zwei bis vier Wochen ausgewechselt und ist zu diesem Zweck mittels eines positiven Kupplungsstückes mit dem am Katheter befindlichen negativen Kupplungsstück zu verbinden. Der die Dialyse-Lösung enthaltende Behälter wird viermal täglich zum Einbringen frischer Dialyse-Lösung in die Bauchhöhle ausgewechselt und ist zu diesem Zweck über einen Schlauch und ein negatives Kupplungsstück lösbar mit einem zweiten am Überleit-Schlauchsystem befindlichen positiven Kupplungsstück lösbar zu verbinden. Alle Kupplungsstücke lassen sich leicht sterilisieren und steril abdecken, so daß eine Kontamination der mit der Dialyse-Lösung in Kontakt kommenden Teile der Vorrichtung durch die notwendigen An- und Abkuppelvorgänge ausgeschlossen ist.

Im Normalfall wird der Behälter nach dem Einfließenlassen der Dialyse-Lösung in die Bauchhöhle ohne Abzukuppeln eingerollt und am Körper befestigt; der Patient kann seine Tätigkeit praktisch uneingeschränkt aufnehmen. Falls das Tragen des leeren Behälters während der Dialysephase trotzdem als Behinderung empfunden wird, ermöglicht die erfindungsgemäße Vorrichtung mittels der

4

0062148

mit passenden Kupplungsstücken ausgerüsteten Verschluß- bzw. Abfluß-Systeme ein risikoloses Ab- und Wiederankuppeln des Behälters.

Die erfindungsgemäße Vorrichtung besteht also aus einem flexiblem Behälter für die Dialyse-Flüssigkeit, dessen Ausflußpartie mit einem Kupplungsstück versehen ist, aus einem zum Überleiten der Dialyse-Flüssigkeit zum Katheter dienenden sterilen Schlauchsystem mit positiven Kupplungsstücken an beiden Enden und dem Katheter, das an seinem frei liegenden Ende ebenfalls ein Kupplungsstück aufweist. Sämtliche Kupplungsstücke sind mit gut passenden, für Mikroorganismen undurchdringbaren Schutzkappen versehen, welche auch bei langer Lagerung die Sterilität des Schlauchsystems garantieren. Die Kupplungsstücke passen perfekt ineinander und gewährleisten dank ihrer spezifischen Ausgestaltung hermetisch dichte Verbindungen sowie ein schnelles und sicheres An- und Abkuppeln.

In der Zeichnung ist ein Ausführungsbeispiel der erfindungsgemäßen Vorrichtung zur kontinuierlichen ambulanten Peritoneal-Dialyse dargestellt, und zwar zeigt

Fig. 1 eine Ansicht des flexiblen Behälters für die Dialyse-Flüssigkeit mit der steril zu kapselnden Ausflußpartie,

Fig. 2 eine Ansicht des zum Überleiten der Dialyse-Flüssigkeit verwendeten Schlauchsystems,

Fig. 3 eine Ansicht des aus der Bauchhöhle des Patienten herausragenden Endes des Katheters,

Fig. 4 eine Ansicht der beim Entleeren der Bauchhöhle verwendeten Abflußleitung,

Fig. 5 die Ansicht eines negativen Kupplungsstückes mit Abdeckkappe für einen sterilen Verschluß,

Fig. 6 die teilweise geschnittene Ansicht einer Abdeckkappe für die sterile Abdeckung eines positiven Kupplungsstückes,

Fig. 7 eine Draufsicht auf eine aus zwei gegeneinander verschwenkbaren Teilen bestehenden geöffneten Schutzkapsel für eine Kupplungsverbindung und

Fig. 8 eine Stirnansicht der Schutzkapsel gemäß Fig. 7

Die Vorrichtung hat einen flexiblen Behälter 1 für die Aufnahme einer sterilen Dialyse-Flüssigkeit bzw. Dialyse- Lösung, die durch einen Schlauch 3 abfließen kann, der am äußeren Ende ein negatives Kupplungsstück 2 aufweist, welches mit einer Kappe 8 steril abgedeckt werden kann. Das im Behälter 1 liegende Ende des Schlauches 3 wird mittels eines lösbaren Stopfens 16 verschlossen. Dieser Stopfen 16 kann durch die flexible Wand des Behälters 1 erfaßt und aus dem Ende des Schlauches 3 herausgezogen werden, sobald die im Behälter 1 befindliche Dialyse- Lösung appliziert werden soll.

Der Behälter 1 ist außerdem mit einem Einlaufstutzen 14 versehen, durch welchen er gefüllt werden kann. Dieser wird nach dem Füllen des Behälters 1 sofort durch eine Schweißstelle 14a hermetisch verschlossen.

Das zum Überleiten der Dialyse-Lösung verwendete Schlauchsystem 21 besteht aus einem flexiblen Schlauch 4, an dessen einen Ende sich ein positives Kupplungsstück 5 befindet, welches in das negative Kupplungsstück 2 des Behälters 1 paßt. Am anderen Ende des Überleit-Schlauches 4 ist ein weiteres positives Kupplungsstück 6 eines Luer-Lock-Verschlusses vorgesehen, das mit einem aus Edelstahl bestehenden negativen Kupplungsstück 9 des Luer-Lock-Verschlusses verbunden werden kann, welches sich am aus der Bauchwand des Patienten herausragenden Ende des Katheters 10 befindet. Das Katheter 10 ist der dritte Teil der Vorrichtung und wird für dauerhaften Verbleib in die Bauchhöhle eines Patienten einoperiert, während die beiden anderen Teile, nämlich der Behälter 1 und das Überleit-Schlauchsystem 21 auswechselbar sind. Der Behälter 1 wird viermal täglich ausgewechselt, während das Überleit-Schlauchsystem 21 alle zwei bis vier Wochen zu erneuern ist.

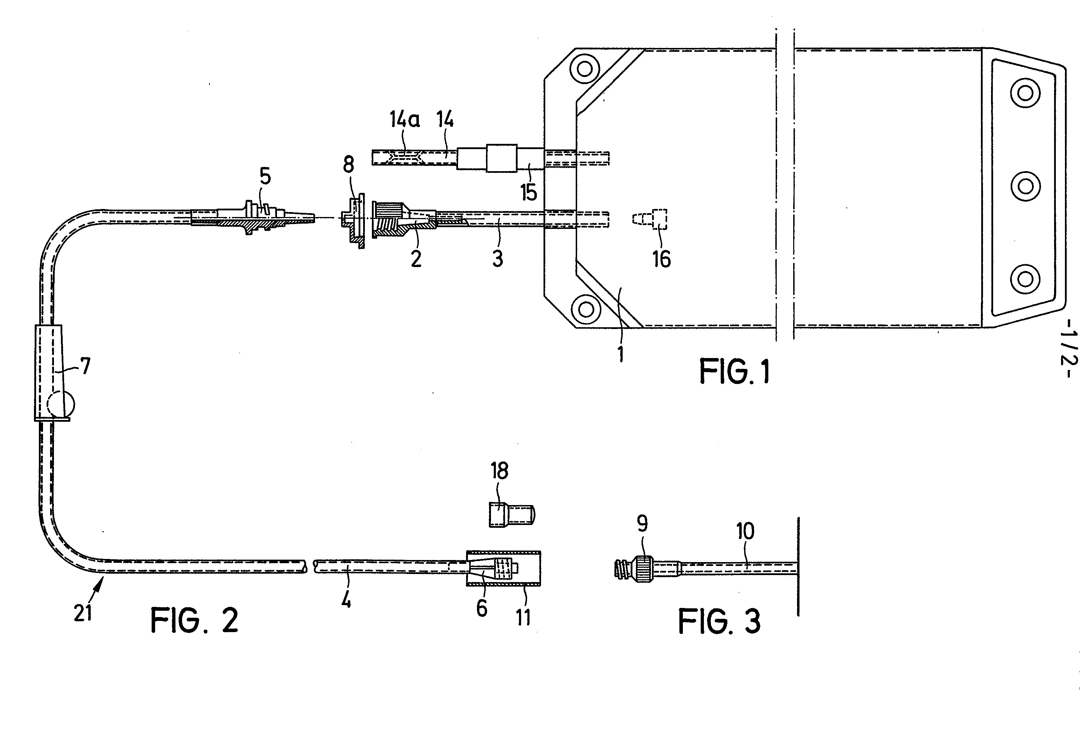

Das Kupplungsstück 5 ist mit einer abnehmbaren Schutzkappe 17 und das Kupplungsstück 6 mit einer ebenfalls abnehmbaren Schutzkappe 18 bakteriendicht abgedeckt. Das gesamte in Fig. 2 gezeigte Überleit-System wird in einer PEEL-Packung (Polyäthylen-Papierpackung) innen und außen steril angeliefert. Hinter dem Kupplungsstück 6 steckt auf dem Silikon-Schlauch 4 ein rohrförmiges Schrumpffolienstück 11. Um das Überleit-Schlauchsystem 21 an das Katheter 10 anzuschließen, wird die Schutzkappe 18 vom Kupplungsstück 6 entfernt, woraufhin man die Kupplungsstücke 6 und 9 miteinander verbindet. Anschließend wird das Schrumpffolienstück 11 über diesen Luer-Lock-Verschluß geschoben und mittels eines Heißluftgebläses aufgeschrumpft, wodurch sich eine hermetische Verbindung ergibt. Diese Verbindung bleibt wochenlang dicht und fest. Zum Abkuppeln des Überleit-Schlauchsystems 21

wird das Schrumpffolienstück 11 zerstört. Das Kupplungsstück 9 besteht aus Edelstahl und hat deshalb eine hervorragende Formstabilität, so daß sich auch bei langjährigem Gebrauch keine Veränderungen ergeben. Das Kupplungsstück 6 besteht aus einem festen, aber trotzdem innerhalb minimaler Grenzen gut anpassungsfähigen Kunststoff, so daß sich ein sehr guter Sitz im negativen Kupplungsstück 9 ergibt. Das aufgeschrumpfte Schrumpffolienstück 11 gewährleistet einen Garantieverschluß, so daß Arzt und Patient jederzeit überprüfen können, ob der Verschluß geöffnet worden ist. Zusätzlich bildet das Schrumpffolienstück 11 eine Sicherung gegen unerwünschtes Lockern des Luer-Lock-Verschlusses und verhindert jede Kontamination und Verschmutzung der Oberfäche der Kupplungsstücke, schützt die betroffene Hautpartie des Patienten vor einem direkten Kontakt mit dem aus Metall bestehenden Kupplungsstück 9 und verhindert dadurch ausgelöste Hautreizungen.

Der Schlauch 4 des Überleit-Schlauchsystems 21 ist ein Silikonschlauch. Auf ihm sitzt eine Rollklemme 7, mit deren Hilfe der Schlauch ganz oder teilweise abgeklemmt werden kann. Der Silikonschlauch gewährleistet ein rasches Einbringen der Dialyse-Lösung in die Bauchhöhle wie auch ein schnelles Entleeren derselben, da er dem Durchfluß der Lösung nur geringen Widerstand entgegensetzt. Im Vergleich zu aus anderen Kunsttoffen gefertigten Schläuchen hat der Silikonschlauch die Vorteile eines geringen Reibungswiderstandes gegenüber der Dialyse-Lösung, so daß wesentlich kürzere Einlauf- und Entleerungszeiten als bei Einsatz von Schläuchen aus Weich-PVC erzielt werden, und eine größere mechanische Festigkeit und bessere Formregenerationsfähigkeit als Schläuche aus Weich-PVC oder Polyäthylen, so daß die Rollklemme 7 keine bleibenden Eindruckstellen erzeugt und dementsprechend das Durchflußlumen nicht unerwünscht verringert werden kann.

Der flexible Behälter 1 muß vom Patienten viermal täglich ausgewechselt werden. Falls der Behälter 1 nach dem Einbringen der Dialyse-Lösung in die Bauchhöhle bis zum Entleerungsvorgang abgekuppelt wird, ist er sogar achtmal täglich an- und abzukuppeln. Beim erstmaligen Anschließen des Behälters 1 an das Schlauchsystem 21 wird wie folgt vorgegangen:

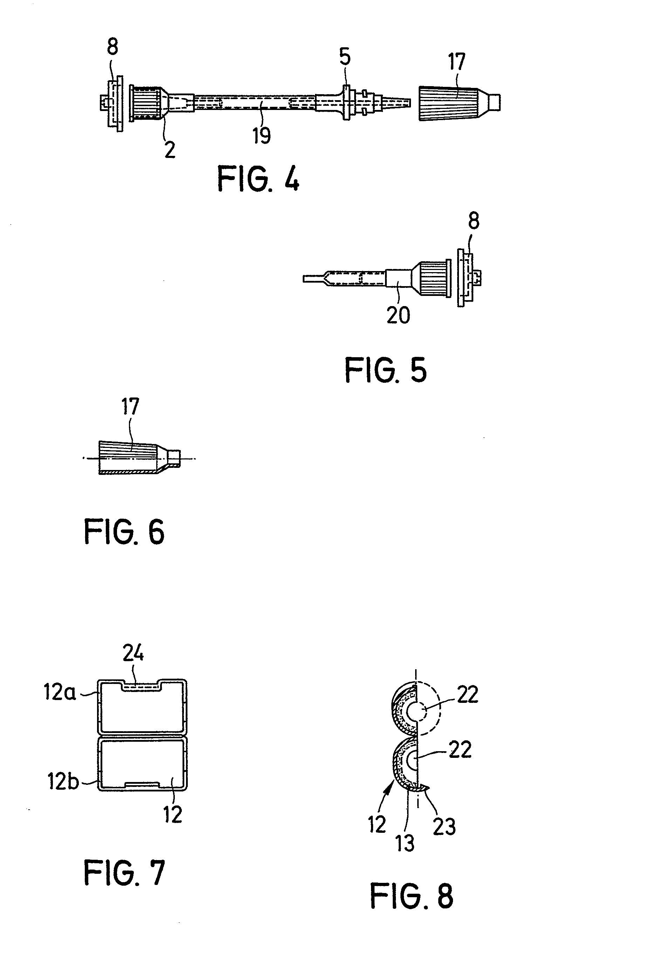

Zunächst wird der mit frischer Dialyse-Lösung gefüllte Behälter 1 bereitgelegt. Dann erfaßt man das am Ende des Schlauches 4 befindliche positive Kupplungsstück 5 beispielsweise mit der linken Hand, entfernt gleichzeitig die Schutzkappe 17 mit der rechten Hand, erfaßt mit dieser nachher das am Schlauch 3 des Behälters 1 befindliche negative Kupplungsstück 2, sprengt durch Druck mit dem Daumen der rechten Hand die Schutzkappe 8 vom Kupplungsstück 2 ab und steckt die beiden Kupplungsstücke 5 und 2 ineinander. Die Kupplungsstücke werden durch Schraubverschluß aneinander fixiert und dann mittels einer zur Desinfektion dienenden Schutzkapsel 12 abgedeckt. Die Schutzkapsel 12 besteht aus zwei gelenkig miteinander verbundenen, im Querschnitt etwa halbkreisförmigen Teilen 12a und 12b, welche an ihren Stirnenden halbkreisförmige Öffnungen 22 zum Durchführen der Schläuche 3 und 4 enthalten. Beide Teile 12a und 12b können mittels einer als Rast ausgebildeten Zunge 23, welche hinter eine Schulter 24 greift, in der in Fig. 8 strichpunktiert dargestellten Lage die Kupplung umschließend zusammengehalten werden.

In der aufklappbaren Schutzkapsel 12 befindet sich eine saugfähige Einlage 13, welche eine ausreichende Menge Desinfektionsflüssigkeit aufnehmen kann, so daß alle

9    0062148

im direkten Kontaktbereich befindlichen Teile der Kupplung zuverlässig desinfiziert werden, wenn die Schutzkapsel 12 geschlossen ist.

Zum Auswechseln des Behälters 1 erfaßt man das Überleit-Schlauchsystem 21 unmittelbar hinter dem Kupplungsstück 5 beispielsweise mit der linken Hand und öffnet und entfernt die Schutzkapsel 12 mit der rechten Hand. Dann wird das Kupplungsstück 2 mit der rechten Hand erfaßt, so daß die Kupplung aufgedreht und gelöst werden kann.

Die Kupplungsstücke 2 und 5 bestehen aus formstabilem hochwertigen Kunststoff, sind großlumig und haben zum Fixieren Schraubgewinde.

Wenn das positive Kupplungsstück 5 nach Entfernen der Schutzkappe 17 oder der Schutzkapsel 12 für einen kurzen Moment nicht mehr zuverlässig gegen Kontamination geschützt ist, kann es an seiner Oberfläche mit Desinfektionsmittel abgespült werden, da dieser Teil des Kupplungsstückes 5 leicht zu reinigen ist.

Dank des großen Lumens aller Kupplungsstücke ist ein rascher Durchfluß der auszutauschenden Dialyse-Lösung gewährleistet.

Die Schutzkappe 8 und die Randpartie des negativen Kupplungsstückes 2 sind derart ausgebildet, daß die Schutzkappe 8 einhändig mittels Daumendruck abgesprengt werden kann. Daher besteht keine Kontaminationsgefahr für das positive Kupplungsstück 5, weil dasselbe zum Öffnen des negativen Kupplungsstückes 2 nicht auf einer Unterlage abgelegt werden muß oder von der Hand des Patienten berührt wird. Die Schraubverbindung der Kupplungsstücke 2 und 5 lockert sich nicht, so daß ein Undichtwerden der Kupplung unmöglich ist und jedes Kontaminationsrisiko ausgeschlossen wird.

Der flexible Behälter 1 ist ein zusammenlegbarer flacher Beutel aus Kunststoff einer als Behältermaterial für Infusionslösungen zugelassenen Spezialqualität. Er hat eine nicht auftragende langgestreckte Form, ist geschmeidig, besteht aus körperfreundlichem Material, hat ein glasklares Aussehen und ist im leeren Zustand gut rollbar. Die Dialyse-Lösung wird ohne Beeinträchtigung der Dichtigkeit des Behälters 1 durch den als Schlauchstück ausgebildeten Einlaufstutzen 14 eingefüllt und letzterer durch Verschweißen (14a) anschließend verschlossen. Die Dialyse-Lösung läßt sich bezüglich der Zusammensetzung dank einer Vorrichtung für die Medikamentenzugabe ohne Beeinträchtigung der Intaktheit des Behälters 1 den laufenden Bedürfnissen anpassen. Die Vorrichtung besteht aus einem auf das Einlaufschlauchstück 14 aufgezogenen, hermetisch dicht aufliegenden Latex-Schlauchstück 15. Die Medikamentenzugabe erfolgt mittels Injektion aus einer Injektionsspritze in die Wand der durch das Latex-Schlauchstück 15 abgedichteten Einlaufschlauchpartie. Nach Herausziehen der Injektionsnadel schließt sich die Einstichstelle in der Latex-Wandung flüssigkeitsdicht, so daß der Behälter 1 während der gesamten Applikationsdauer trotz der Medikamentenzugabe dicht bleibt.

Das in Fig. 4 dargestellte Abflußleitungssystem wie auch das Verschlußsystem der Fig. 5 ermöglichen das temporäre Abkuppeln des entleerten Behälters 1 nach dem Einfließenlassen der Dialyse-Lösung vom Überleit-Schlauch 21 gemäß Fig. 2 sowie ein erneutes Ankuppeln des leeren Behälters für den Rücklauf der Lösung aus der Bauchhöhle ohne jedes Kontaminationsrisiko. Die in Fig. 4

0062148

dargestellte Abflußleitung 19 ist an beiden Enden mit einem negativen Kupplungsstück 2 und einem positiven Kupplungsstück 5 versehen, welche mittels Schutzkappen 8 bzw. 17 steril verschlossen sind. Die Kupplungsstücke 2 und 5 sind ebenso wie die an den Schläuchen 3 und 4 angebrachten Kupplungsstücke 2 und 5 ausgebildet. Auch das in Fig. 5 dargestellte Verschlußsystem 20 enthält als Kupplungselement das negative Kupplungsstück 2. Daher kann man den Behälter 1 nach Applikation der in ihm enthaltenen Dialyse-Lösung abkuppeln und das Kupplungsstück 5 mit dem Verschlußsystem 20 (Fig. 5) steril verschließen. Zum Entleeren der Bauchhöhle des Patienten wird das Verschlußsystem 20 wieder abgekuppelt und durch die Abflußvorrichtung gemäß Fig. 4 ersetzt, wobei der leere Behälter 1 an das Kupplungsstück 5 angeschlossen wird.

Patentansprüche:

1. Vorrichtung zur kontinuierlichen ambulanten Peritoneal-Dialyse (CAPD), mit einem flexiblen Behälter für die Dialyse-Flüssigkeit, einem Überleit-Schlauchsystem und einem abkuppelbaren Katheter, dadurch gekennzeichnet, daß das Überleit-Schlauchsystem (21) sowohl vom Behälter (1) als auch vom Katheter (10) abkuppelbar ist und daß die im gesamten Schlauchsystem vorgesehenen Kupplungsstücke (2, 5, 6) mittels Schutzkappen (8, 17, 18) steril abzudecken sind.

2. Vorrichtung nach Anspruch 1, dadurch gekennzeichnet, daß das Überleit-Schlauchsystem (21) einen Silikon-Schlauch (4) aufweist, der an beiden Enden mit einem positiven Kupplungsstück (5, 6) versehen ist, das in ein negatives Kupplungsstück (2, 9) am Behälter (1) bzw. Katheter (10) paßt.

3. Vorrichtung nach Anspruch 1 oder 2, dadurch gekennzeichnet, daß für jedes positive Kupplungsstück eine auf dasselbe aufsteckbare, leicht zu entfernende Schutzkappe (17, 18) vorgesehen ist.

4. Vorrichtung nach Anspruch 1 oder 2, dadurch gekennzeichnet, daß das negative Kupplungsstück (2) mit einer durch einfachen Daumendruck abzusprengenden und somit einhändig zu entfernenden Schutzkappe (8) versehen ist.

2

0062148

5. Vorrichtung nach einem der Ansprüche 1 bis 4, dadurch gekennzeichnet, daß die zusammengesteckten Kupplungen (2, 5 und 6, 9) jeweils mit einer Schutzkapsel (12) bzw. einer Schrumpfhülle (11) zu versehen sind.

6. Vorrichtung nach Anspruch 5, dadurch gekennzeichnet, daß die Schrumpfhülle (11) aus einem unter Wärmeeinfluß schrumpfenden Schlauchstück besteht.

7. Vorrichtung nach Anspruch 5, dadurch gekennzeichnet, daß die Schutzkapsel (12) ein zu öffnendes Desinfektionsbehältnis ist, das eine mit Desinfektionsflüssigkeit getränkte Einlage (13) enthält.

8. Vorrichtung nach einem der Ansprüche 1 bis 7, dadurch gekennzeichnet, daß die Kupplungen (2, 5 und 6, 9) Luer-Lock-Kupplungen sind.

14a    14

5    8

15

2    3

1    16

FIG. 1

7

18

9    10

4    6    11

21    FIG. 2    FIG. 3

-1/2-

0062148

0062148

**FIG. 4**

**FIG. 5**

**FIG. 6**

**FIG. 7**

**FIG. 8**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y,A | FR-A-2 455 462 (SMAD)<br><br>* Figur 2; Seite 5, Zeilen 1-4, 13-15; Seite 6, Zeilen 5-10 *<br><br>--- | 1,3,5 7 | A 61 M 1/03<br>A 61 M 25/00 |
| Y | US-A-3 976 311 (SPENDLOVE)<br>* Figur 2; Zusammenfassung *<br><br>--- | 1,3,5 | |
| A | DE-A-2 809 303 (POPOVICH)<br>* Seite 13, Zeilen 12-14 *<br><br>--- | 2 | |
| A | GB-A-2 021 418 (BAXTER)<br>* Figur 1; Bezugszeichen 44 *<br><br>--- | 4 | |
| A | US-A-3 648 693 (TERUMO)<br>* Spalte 2, Zeilen 25-28 *<br><br>--- | 5,6 | |
| A | DE-A-2 853 635 (AFFELD)<br>* Anspruch 4 *<br><br>--- | 7 | RECHERCHIERTE SACHGEBIETE (Int. Cl. ³)<br><br>A 61 M |
| A | US-A-2 040 379 (BAXTER)<br>* Zusammenfassung *<br><br>--- | 8 | |
| A | GB-A-2 000 685 (JOHNSON)<br><br>* Figur 2 *<br><br>----- | | |

Der vorliegende Recherchenbericht wurde für alle Patentansprüche erstellt.

| Recherchenort | Abschlußdatum der Recherche | Prüfer |
|---|---|---|
| DEN HAAG | 21-06-1982 | PETZUCH M. |